# EUROPÄISCHE PATENTANMELDUNG

(11) **EP 0 763 363 A1**
(43) Veröffentlichungstag der Anmeldung: **19.03.1997**
(21) Anmeldenummer: 96113876.5
(22) Anmeldetag: 29.08.1996
(51) Int. Cl.: A61L 2/26, G01N 31/22

(54) **Vorrichtung zum Kontrollieren und Testen von nach dem Vakuumverfahren arbeitenden Dampsterilisatoren**

(30) Priorität: 30.08.1995 DE 29513992 U
(71) Anmelder: Schefter, Siegfried, 41516 Grevenbroich (DE)
(72) Erfinder: Schefter, Siegfried, 41516 Grevenbroich (DE)

(57) **Zusammenfassung**

Dampfsterilisatoren werden üblicherweise mit chemischen Farbindikatoren auf ihre Wirksamkeit geprüft. Dazu werden Prüfkörper bzw. -systeme mit integrierten Indikatoren in einer Sterilisierkammer einem Sterilisationsprozeß unterworfen. Aufgabe der Erfindung ist, eine Vorrichtung zu schaffen, die keine verfälschten Test- und Kontrollergebnisse liefern kann.

Dies wird dadurch erreicht, daß im Zentrum eines Prüfmaterialstapels (1) aus gewebten oder ungewebten, porösen hydrophilen oder hydrophoben Flächengebilden (11,12,15,16,17,18) ein chemischer Farbindikator (4) sandwichartig zwischen zwei gleichförmigen, sich gegenüberliegenden wärmeisolierenden Raumgebilden (13,14) angeordnet wird. Die Wärmeisolierung bewirkt, daß schädliche Luft während der Sterilisierphase länger als üblich im Indikatorbereich verharrt und einen zum Farbumschlag führenden Dampfkontakt verhindert.

## Beschreibung

Die Erfindung betrifft ein Verfahren sowie einen Prüfmaterialstapel aus gewebten oder ungewebten, porösen Flächengebilden zum Kontrollieren und Testen von nach dem Vakuumverfahren arbeitenden Dampfsterilisatoren, in dessen Zentrum zwischen 2 gleichförmigen, sich gegenüberliegenden, wärmeisolierenden Raumgebilden ein chemischer Farbindikator eingelegt wird.

Prüfmaterialstapel zum Testen von Dampfsterilisatoren sind in mannigfacher Form und Ausführung bekannt. Neben dem weltweit bekannten Bowie & Dick-Testpaket aus Baumwolltüchern (Bowie et al: The Lancet, March 16, 1963, 586 - 587) sind alternative Teststapel (Testpakete) aus ungewebten, porösen hydrophilen und hydrophoben Materialien bekannt. Dabei handelt es sich um einmal- und wiederverwendbare Testmaterialstapel in vorgeschriebenen Zusammenhaltemitteln (US 4,486,387; US 4,576,795; US 4,579,715; US 4,596,696; US 4,692,307; US 4,699,765; US 5,200,147; GB 2 180 933 A; EP 0 233 226 B1; DE 94 01 058; G 92 06 918.5). Alle diese vorgenannten Testmaterialstapel sind nur dazu geeignet, Dampfsterilisatoren mit einem speziellen Testprogramm (Bowie&Dick-Test) in unbeladenem Zustand auf ihre Wirksamkeit zu prüfen. Die Auskunft darüber gibt ein mit einem chemischen Farbindikator bedruckter Testbogen, der mittig im Testpaket liegend bei Sattdampfkontakt von 134 Grad Celsius in einem Zeitraum von 2-3 Minuten einen Farbumschlag zeigen soll. Bestimmte von den jeweiligen Herstellern der Testbögen genannte Kriterien sagen aus, ob das Gerät einwandfrei arbeitet oder Mängel hat.

Sterilisatoren in Krankenhäusern arbeiten überwiegend nach dem fraktionierten Vakuumverfahren. Grundlage für die Herbeiführung von Sterilität am und im zu sterilisierenden Gut ist eine ausreichende Luftentfernung und das anschließende Einwirken von feuchter Wärme (Sattdampf), wobei im Bereich einer Luftinsel der Dampf keine sterilisierende Wirkung ausüben kann, da sich Luft und Dampf grundsätzlich nicht vermischen. Hauptproblem ist die sog. schleichende Verschlechterung des Vakuums. Um dieser gefährlichen Entwicklung frühzeitig auf die Spur zu kommen, wird der sog. Bowie&Dick-Test durchgeführt. Dabei liegt der Testmaterialstapel in der ansonsten leeren Kammer und die Einwirkzeit der Arbeitstemperatur von 134 Grad C beträgt maximal 3,5 Minuten. Wird die Zeit überschritten, ist das Testergebnis - besonders wenn es keinen Defekt anzeigt - nicht brauchbar. Ziel dieser praxisuntypischen Bedingungen (Teilbeladung der Sterilisierkammer, sehr kurze Einwirkzeit des Dampfes) ist, die Effizienz der Vakuumeinrichtung zu prüfen und die Verschleierung eines vorhandenen Defekts auszuschließen.

Luft, die sich nach Beendigung der Vakuumphase durch unzureichend erzeugtes Vakuum oder eine Leckage am Sterilisator in der Kammer befindet, oder nichtkondensierbare Gase, die mit dem Dampf in die Kammer gelangen, wird von dem Testmaterialstapel aufgesogen und flüchtet vor dem von allen Seiten in den Teststapel eindringenden Dampf und die rundherum ansteigende Temperatur ins Zentrum, wo es vorläufig noch kälter ist. Durch die fast leere Kammer wird im Testmaterialstapel in relativ kurzer Zeit die Arbeitstemperatur von 134 Grad Celsius erreicht, nur nicht in der Luftblase. Aufgrund der schlechten Wärmeleitfähigkeit von Luft herrscht innerhalb einer Luftblase ein starkes Temperaturgefälle von außen nach innen. Wenn die Ränder der Luftblase die Umgebungstemperatur angenommen haben, strömt die aufgeheizte Luft aus dem Testmaterialstapel, was zur Folge hat, daß die Luftmenge im Zentrum des Stapels stetig abnimmt, Dampf den Farbindikator nachträglich umschlagen läßt und damit das Testergebnis verfälscht.

Viel gravierender sind jedoch die Folgen einer Verfälschung bei der Chargenkontrolle. In Krankenhäusern werden zum Nachweis einer sicheren sterilen Versorgung der Patienten zunehmend Kontrollen der Sterilisierchargen des täglich benötigten Sterilgutes durchgeführt und dokumentiert. Dazu werden sog. Sterilisiergutsimulatoren zum zu sterilisierenden Gut gelegt und mitsterilisiert. Die bekannten und bereits erwähnten Testmaterialstapel sowie Helixe erfüllen die Anforderungen in Verbindung mit Indikatoren für den Bowie&Dick-Test nicht. Denn anders als beim Testen sind hierbei 2 Bedingungen anders:
1. Die Sterilisierkammer ist voll beladen.
2. Die Einwirkzeit (Sterilisierzeit) des Dampfes auf das Gut beträgt mindestens 5 Minuten bei der Arbeitstemperatur von 134 Grad Celsius.

Dazu kommt, daß die Temperatur in allen bekannten Kontrollsystemen ohne Verzögerung auf den Indikatorbereich einwirken kann. Das hat zur Folge, daß die längere Steige- und Sterilisierzeit die nachträgliche Austreibung schädlicher Luft aus dem Testmaterialstapel intensiviert, wobei die Luftaustreibungsgeschwindigkeit in ungewebtem, porösem Material höher ist als in gewebtem, porösem Material.

Der in Schutzanspruch 1 angegebenen Erfindung liegt das Problem zugrunde, einen Prüfmaterialstapel zu gestalten, der in Verbindung mit Farbindikatoren für den Bowie&Dick-Test bei vollgeladener Sterilisierkammer und Sterilisierzeiten von 5 Minuten und länger eine nachträgliche Luftaustreibung aus dem Prüfmaterialstapel so lange hinauszögert, daß die Aussage des chemischen Farbindikators nicht verfälscht wird. Dieses Problem wird mit den in den Schutzansprüchen 1, 2, 3 und 5 aufgeführten Merkmalen gelöst.

Mit der Erfindung wird erreicht, daß sich die während der Steige-, Ausgleich- und Sterilisierzeit in der mit zu sterilisierendem Gut gefüllten Sterilisierkammer befindliche schädliche Luft oder Inertgase u.a. im Zentrum zwischen den beiden wärmeisolierenden Raumgebilden (nachfolgend "Inlays" genannt) in den dort angeordneten porösen Flächengebilden (nachfolgend "Lagen" genannt) des Prüfmaterialstapels und dem Indikator einnistet und dort wegen der zeitlich stark verzögerten Aufheizung des Inlay-Werkstoffs gegenüber der sonstigen Umgebung länger als gewöhnlich verharrt. Dadurch wird ein ausreichender Dampfkontakt mit dem Indikator verhindert, der durch den unzureichenden Farbumschlag den fehlerhaften Prozeßverlauf und damit die Sterilisierunsicherheit für das in der Kammer befindliche Gut signalisiert.

Ein weiterer Vorteil liegt in der noch genaueren Aussage bei der Verwendung des Prüfmaterialstapels für den Bowie&Dick-Test.

Der in der Erfindung aufgeführte Prüfmaterialstapel hat auch den Vorteil, daß der Bowie&Dick-Test nun auch in älteren Sterilisatoren durchgeführt werden kann, die kein spezielles Testprogramm besitzen. Diese können dann mit dem Standard-Wäscheprogramm getestet werden mit Sterilisierzeiten, die deutlich über 3,5 Minuten liegen.

Desweiteren ist ein Vorteil, daß der Prüfmaterialstapel als Einmalpack in Sterilisationspapier oder -vliess eingeschlagen und mit Klebeband gesichert werden kann. Zur Wiederverwendung wird der Prüfmaterialstapel in eine enganliegende Einsteckkassette eingeschoben.

Der Prüfmaterialstapel besteht vorteilhafterweise aus vielen Lagen saugfähigen Filtrierkartons. Die Höhe des Prüfmaterialstapels richtet sich nach der Dichte des Materials und der Anforderung an seinen kritischen Zustand. Im vorliegenden Ausführungsbeispiel hat der Prüfmaterialstapel eine Abmessung von 150 x 115 x 34 mm (L/B/H). Die im Zentrum sich gegenüberliegenden, in Vertiefungen der beiden Stapelhälften einliegenden Inlays mögen aus Silikon oder Keramik bestehen und sollen, um die Homogenität des Stapels weitgehend zu erhalten, die Vertiefungen gänzlich ausfüllen. Die Maße der Inlays sind je 60 x 25 x 5 mm (L/B/H). Die zwischen den Inlays angeordneten Lagen Filtrierkartons sind möglichst dünn, in diesem Fall je ca. 2 mm, und bringen damit die Inlays sehr dicht aneinander, was dazu führt, daß die Temperatur zwischen ihnen länger niedriger bleibt als im übrigen Prüfmaterialstapel. Der Indikatorbogen ist sandwichartig zwischen den Lagen und Inlays eingebettet. Die flächenmäßige Ausdehnung der Inlays bestimmt die Größe des Areals auf dem Indikatorbogen, das Defekte anzuzeigen vermag; die Dicke der Inlays beeinflußt die Geschwindigkeit des Temperaturanstiegs zwischen den Inlays und den Zeitpunkt des Erreichens der Arbeitstemperatur (Sterilisiertemperatur). Beide Maße können nach der Klasse des Indikators und dem gewünschten Wirkungsgrad des Prüfmaterialstapels bestimmt werden.

Die beiden Stapelhälften des Prüfmaterialstapels können statt aus mehreren Lagen ungewebtem, porösem Materials je aus einem ganzstückigen Block des Materials mit einer entsprechenden Vertiefung für das Inlay bestehen.

Die Erfindung wird nachfolgend anhand des in den Figuren 1 und 2 dargestellten Ausführungsbeispiels und durch Temperatur/Zeit-Diagramme in den Figuren 3, 4 und 5 erläutert. Es zeigen:

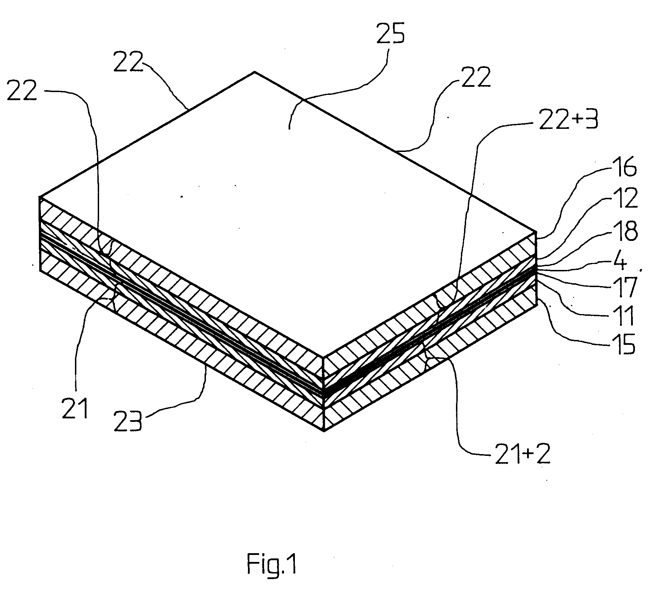

- Fig. 1: den Prüfmaterialstapel in einsatzbereitem Zustand von schräg oben, jedoch ohne Zusammenhaltemittel;

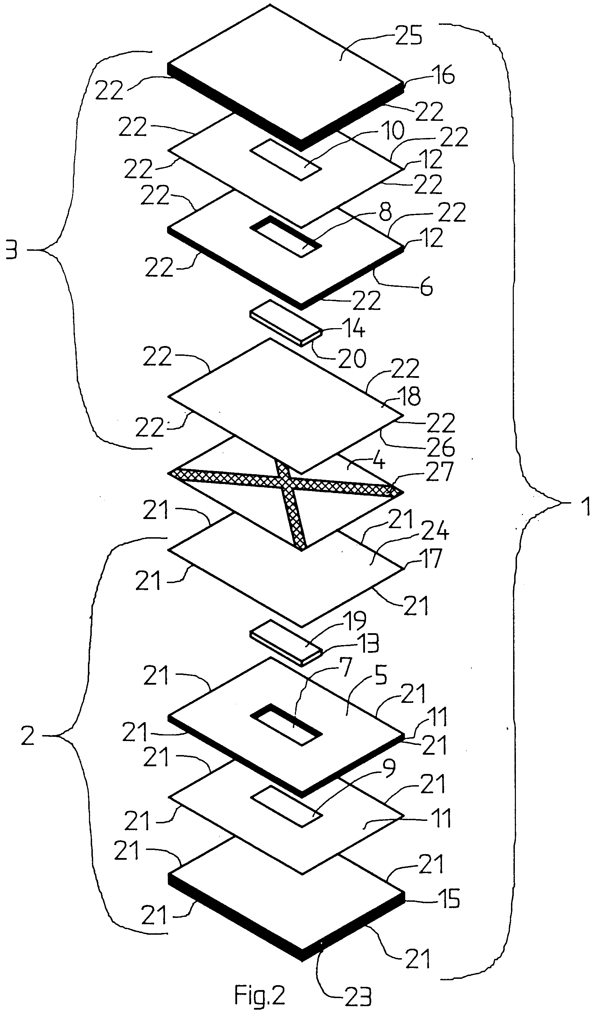

- Fig. 2: den Prüfmaterialstapel als Explosionsdarstellung von schräg oben;

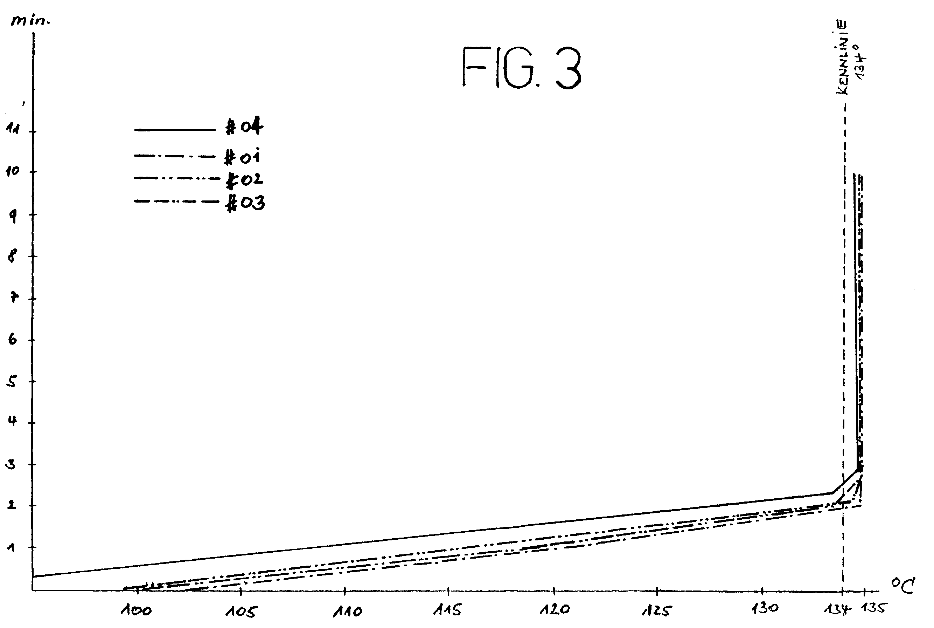

- Fig. 3: Temperatur/Zeit-Diagramm über die Temperaturentwicklung in der Strömungsleitung einer Sterilisierkammer, eines Testmaterialstapels aus gewebtem, porösem hydrophilem Material (Baumwolle), je eines Prüfmaterialstapels aus ungewebtem, porösem hydrophilem Material mit bzw. ohne wärmeisolierenden Inlays bei fehlerlos verlaufendem Sterilisierprogramm;

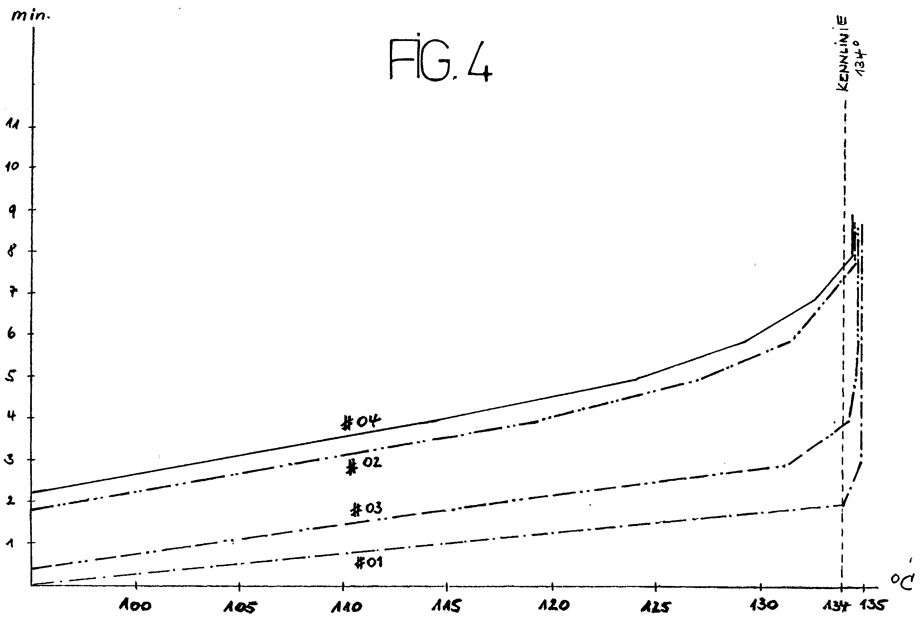

- Fig. 4: Temperatur/Zeit-Diagramm wie Fig. 3, jedoch mit ungenügendem Vorvakuum;

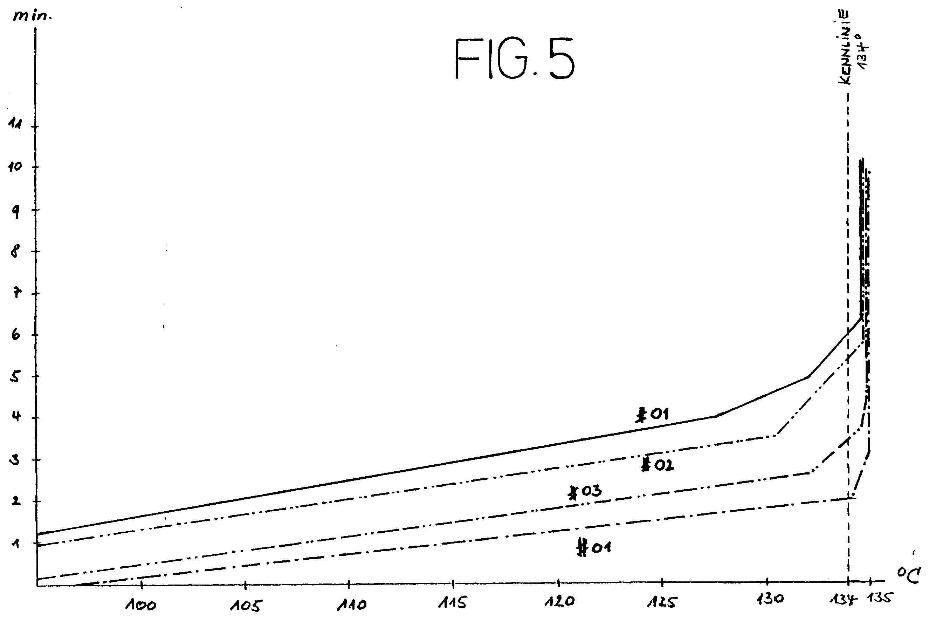

- Fig. 5: Temperatur/Zeit-Diagramm wie Fig. 3, jedoch mit undichter Sterilisierkammer (Leckage).

In den Figuren 1 und 2 ist der Prüfmaterialstapel 1, bestehend aus den beiden identischen Stapelhälften 2 und 3, dargestellt. Die gleichgroßen Flächengebilde 11,12,15,16,17 und 18 liegen bündig aufeinander, wobei die Lagen 11 und 12 einen gleichförmigen und gleichgroßen Ausschnitt 9 und 10 besitzen und zusammengefügt je eine Vertiefung 7 und 8 zur Aufnahme des wärmeisolierenden Raumgebildes 13 und 14 besitzt. Die Anzahl der Lagen 11 und 12 richtet sich nach der Dicke der Inlays 13 und 14, beide sind gleich hoch. Je ein Flächengebilde 17 und 18 schließt die Stapelhälften 2 und 3 zum Zentrum des Prüfmaterialstapels 1 ab. Zwischen beiden Stapelhälften 2 und 3 ist ein Indikatorbogen 4 angeordnet.

Die Figuren 3, 4 und 5 zeigen je 1 Diagramm über die Temperaturentwicklung an mehreren Meßpunkten in einer Sterilisierkammer in Korrelation mit der Zeit während eines Sterilisationsprozesses mit
* einwandfreien Bedingungen (Fig. 3),
* verschlechtertem Vakuum (Fig. 4),
* undichter Kammer (Fig. 5).

Das heißt, daß sich bei den Figuren 4 und 5 bei Beginn der Sterilisierphase erhebliche Mengen schädlicher Luft in den Test- und Prüfmaterialstapeln befinden. Die Temperaturen werden mit Thermoelementen gemessen.
Die Temperaturkurven werden wie folgt zugeordnet:
- #01: Strömungsleitung (Kammerabfluß) in der Sterilisierkammer,
- #02: Zentrum eines Testmaterialstapels aus Baumwolltüchern nach EN 285,
- #03: Zentrum eines Prüfmaterialstapels aus ungewebtem, porösem hydrophilem Material ohne wärmeisolierende Inlays,
- #04: Zentrum eines Prüfmaterialstapels aus ungewebtem, porösem hydrophilem Material mit wärmeisolierenden Inlays.

Entscheidend für die Beurteilung der Diagramme ist der Zeitpunkt, zu dem die Temperaturkurve der Thermoelemente #02, #03 und #04 die des Thermoelements #01 tangiert. Das ist der zuvor beschriebene Moment, in dem am Meßpunkt die vorher angesiedelte schädliche Luft ausgetrieben ist und durch Dampf ersetzt wird. Damit ist das Kontrollergebnis verfälscht.

## Patentansprüche

1. Vorrichtung zum Kontrollieren und Testen von Dampfsterilisatoren mit 2 Stapelhälften aus gewebten oder ungewebten, porösen hydrophilen oder hydrophoben Flächengebilden zum ein- oder mehrmaligen Gebrauch in dem jeweils dafür vorgeschriebenen oder vorgesehenen Zusammenhaltemittel, zwischen deren einander zugewandten Flachseiten ein Indikator anzuordnen ist, dadurch gekennzeichnet, daß jede der beiden Stapelhälften (2,3) im Zentrum der dem Indikator (4) zugewandten Fläche (5,6) eine Vertiefung (7,8) durch einen gleichförmigen und gleichgroßen Ausschnitt (9,10) in mehreren aneinanderliegenden Flächengebilden (11,12) aufweist, die je durch ein einstückiges, wärmeisolierendes Raumgebilde (13,14) aus gas- und flüssigkeitsundurchlässigem Werkstoff mit einem wesentlich geringeren Wärmeleitwert als dem der Flächengebilde (11,12,15,16,17,18) gänzlich ausgefüllt wird, und daß nach dem Zusammenfügen der beiden Stapelhälften (2,3) zum Prüfmaterialstapel (1) die sichtbaren Flächen der beiden einstückigen Raumgebilde (19,20) deckungsgleich aufeinanderliegen.

2. Vorrichtung nach Anspruch 1, dadurch gekennzeichnet, daß die dem Indikator (4) zugewandten Oberflächen (19,20) der einstückigen Raumgebilde (13,14) bündig mit den dem Indikator zugewandten Oberflächen (5,6) der ihnen zugehörigen, zusammengefügten Flächengebilde (11,12) abschließen.

3. Vorrichtung nach Anspruch 1, dadurch gekennzeichnet, daß zwischen dem Indikator (4) und den je mit einem einstückigen Raumgebilde (13, 14) ausgefüllten Stapelhälften (2,3) mindestens je ein keinen Ausschnitt aufweisendes Flächengebilde (17,18) des Prüfmaterialstapels (1) angeordnet ist.

4. Vorrichtung nach Anspruch 3, dadurch gekennzeichnet, daß zur Markierung der Mitte des Prüfmaterialstapels (1) die unmittelbar am Indikator (4) anliegenden beiden keinen Ausschnitt aufweisenden Flächengebilde (17,18) einen anderen Farbton besitzen als die übrigen Flächengebilde (11,12,15,16,17,18) des Prüfmaterialstapels (1).

5. Vorrichtung nach den Ansprüchen 1 und 2, dadurch gekennzeichnet, daß die Ausdehnung der beiden einstückigen Raumgebilde (13,14) zu den 4 Seitenflächen (21,22) und der beiden je dem Indikator (4) zu- und abgewandten Flächen (23,24,25,26) der beiden Stapelhälften (2,3) höchstens so groß ist, daß bei zusammengefügten Stapelhälften (2,3) zum Prüfmaterialstapel (1) an allen 6 Flächen (21,22,23,24) das Material der Flächengebilde (11,12,15,16,17,18) ununterbrochen sichtbar ist.

6. Vorrichtung nach den Ansprüchen 1, 2 und 5, dadurch gekennzeichnet, daß das einstückige Raumgebilde (13,14) jede geometrische Form aufweisen kann.

7. Vorrichtung nach den Ansprüchen 1 und 3, dadurch gekennzeichnet, daß sich der zwischen den beiden Stapelhälften (2,3) angeordnete Indikator (4) über die gesamte Flächengebildefläche (27) erstreckt.

8. Vorrichtung nach den Ansprüchen 1 und 7, dadurch gekennzeichnet, daß sich der zwischen den beiden Stapelhälften (2,3) angeordnete Indikator (4) nur über einen Teil der Flächengebildefläche (27) erstreckt, wobei der Indikator mindestens den gesamten Flächenbereich (19,20) der beiden einander deckungsgleich zugewandten Raumgebilde (13,14) bedeckt.
